# EUROPEAN PATENT APPLICATION

(11) **EP 1 217 360 A2**
(43) Date of publication of application: **26.06.2002**
(21) Application number: 01830779.3
(22) Date of filing: 19.12.2001
(51) Int. Cl.: G01N 27/00, G01N 29/02, G01N 33/00, G01N 33/18

(54) **Use of styrene based syndiotactic polymers as sensing elements for detection of organic pollutants**

(30) Priority: 20.12.2000 IT SA000023
(71) Applicant: Universita' Degli Studi di Salerno, 84084 Fisciano (SA) (IT)
(72) Inventor: Guerra, Gaetano, 84100 Salerno (IT); Mensitieri, Giuseppe, 80132 Napoli (IT); Venditto, Vincenzo, 80131 Napoli (IT)
(74) Representative: Bazzichelli, Alfredo

(57) **Abstract**

The use of the crystalline nanoporous form δ of syndiotactic homopolymer or copolymers of styrene as sensing elements for detection of organic pollutants is disclosed as well as sensors using such polymers. The sensors so obtained are suitable for the detection in air or water of organic pollutants.

## Description

The present invention relates to the use of syndiotactic polystyrene or syndiotactic copolymers of styrene, either at least in part in the crystalline nanoporous form, as sensing elements for detection of organic pollutants in air and water and to sensors using such polymers.

### Background of the invention

Polymeric films are frequently used as sensing elements in sensorics since they offer a lot of advantages: they are generally low cost materials; their fabrication techniques are simple (not involving special clean-room or high temperature processes) and can be deposited on various types of substrates. In fact, polymeric sensing materials have been used for impedance, semiconductor, resonant, electrochemical, calorimetric and fiber optic sensors. In several cases polymer coatings are used which sorb molecules (adsorption on the surface or absorption into the bulk of the material) from the environment.

However, polymeric sensing elements generally present a very poor selectivity between different molecules, since sorption involves amorphous phases.

Widely used are resonant sensors for which the mass increase of the sensing film due to sorption of the analyte from the environment produces a shift of the resonant frequency. Particularly used for the online detection of organic compounds are quartz-crystal-microbalance (QCM) sensors. It is well known that, for this technique, rubbery polymers exhibit a much higher sensitivity, but suffer from viscoelastic influences on the sensor response which are hard to manage if the coating thickness exceeds a substance dependent value. On the other hand, glassy polymers (like atactic polystyrene) behave like a rigid material, but neither offer high sensitivity or fast sensor responses.

There was therefore a need in the art to provide polymeric materials, to be used as sensing materials, not suffering from viscoelastic influences while maintaining high sensitivity and fast responses.

It has now surprisingly found that syndiotactic polystyrene styrene polymers or syndiotactic copolymers of styrene, either at least in part in the nanoporous crystalline δ form, may be used as sensing elements in sensors for the detection of organic pollutants.

It is therefore an object of the present invention the use of syndiotactic polystyrene or of syndiotactic copolymers of styrene, either at least in part in the crystalline nanoporous δ form, as sensing elements in sensors for the detection of organic pollutants.

The present description contains three drawings showing:
Fig.1 a graph reporting the frequency variation of the measurement apparatus associated with instantaneous variations of chloroform pressure versus time;
Fig.2 A and Fig.2 B two graphs reporting the frequency variation of the measurement apparatus associated with instantaneous variations of chloroform pressure versus time, at 35°C and 56°C, respectively.

Syndiotactic polystyrene is a rigid semicrystalline material with a glass transition temperature close to 80-90°C and a melting temperature close to 270°C which presents several crystalline forms. By syndiotactic styrene polymer is meant the polymer where the fraction of syndiotactic (also indicated by *r*) dyads is larger than 0.9. This polymer may be obtained according to the method described in European patent application n.0271875.

Samples including syndiotactic polymers in the nanoporous crystalline δ form, described in the Italian patents IT 1271842 and 1306004 are able to readily absorb, mainly in the crystalline phase rather than in the amorphous phase, suitable volatile organic compounds.

According to the invention may be used syndiotactic styrene homopolymer (syndiotactic polystyrene) or syndiotactic styrene copolymers with CH₂=CH-R olefins, where R is an alkyl-aryl or an halogen-aryl radical with 6-20 carbon atoms, or with other ethylenically unsaturated monomers, which can be copolymerized with styrene. In said copolymers the syndiotactic fraction is likewise larger than 0.9 and the copolymers are crystallizable in the nanoporous δ form. The comonomers may be present in an amount lower than 80% by mol.

The presence of the δ form is essential for the working of the invention. In particular, in the most preferred embodiments of the sensing elements the nanoporous δ form should constitute at least 80% of the syndiotactic styrene polymer or copolymer crystalline fraction.

The nanoporous δ form, as described in the Italian patent IT 1271842, is characterized by X-ray diffraction reflections of higher intensity at 2ϑ (CuKα) ≈ 8.4°, 10.6°, 13.3°, 16.8°, 20.7° 23.5°, and by an intensity ratio between two reflections presenting Miller indexes (010) and (-210), that is I(8.4)/I(10.6), larger than 5. The nanoporous δ form may be produced according the teaching of IT 1271842 and 1306004.

The organic pollutants which can be detected by sensing elements based on syndiotactic styrene polymers according to the present invention, are those which give rise to clathrate structures, the formation of which provoke substantial decreases of the intensity ratio I(8.4)/I(10.6) of X-ray diffraction spectra to values lower than 4. In general terms, according to the invention organic pollutants with molecular dimensions lower than 200 Å³ may be detected. In particular, organic pollutants which can be detected by these sensing elements can be halogenated compounds (like e.g., chloroform, methylene chloride, carbon tetrachloride, dichloroethane, trichloroethylene, tetrachloroethylene, dibromoethane, methylene iodide, chlorobenzene, dichlorobenzene, etc.), aromatic compounds (like e.g., benzene, toluene, styrene, naphthalene, xylene, ethylbenzene, etc.), cyclic compounds (like e.g., cyclohexane, cyclohexene, decalin, tetrahydrofurane, cyclopentanone, etc.) as well as ketones, organic esters and ethers and organic compounds containing sulfur atoms (e.g.,carbon disulfide). It is worth noting that, sensors produced using syndiotactic styrene polymers or copolymers in the nanoporous δ form according to the present invention, are sensitive to the volatile organic compounds most frequently present in industrial wastes, like benzene, toluene, chloroform, methylene chloride, tetrachloroethylene, trichloroethylene and chlorobenzenes. The liquid and gaseous mixtures from which these compounds can be detected can be based on water and air.

Sensing elements for detection of organic pollutants which are based on the crystalline nanoporous form of syndiotactic styrene homopolymer or copolymers present, with respect to those based on other rigid (semicrystalline or glassy) polymers, a higher sensitivity and selectivity, while with respect to those based on rubbery polymers a higher selectivity associated with a substantial absence of viscoelastic influences on the sensor response.

Further objects of the present invention are sensors for the detection of organic pollutants including sensing elements comprising syndiotactic polystyrene or syndiotactic copolymers of styrene, either at least in part in the nanoporous crystalline δ form.

The devices where these sensing elements can be used can be of several different kinds, such as for instance resonant sensors (which measure the shift of the resonance frequency of crystals); impedance type sensors (which measure capacitance and/or resistance changes); electrochemical cells (potentiometric, amperometric and conductometric devices); calorimetric sensors (which measure temperature differences) ; fibre optic sensors (based on changes of the light propagation, absorption and/or emission).

The sensing elements of the present invention are particularly useful in resonant sensors, which can be classified depending on the used waves as bulk acoustic wave (BAW), surface acoustic wave (SAW) and flexural plate wave (FPW). In fact, for such sensors, sensing elements based on syndiotactic polystyrene also present the advantage of a high rigidity, which eliminates possible viscoelastic effects.

A further object of the present invention is a process for the preparation of sensing elements to be used in sensors for the detection of organic pollutants through deposition on the sensor substrate of a polymeric film characterised by the fact that syndiotactic polystyrene or syndiotactic copolymers of styrene, either at least in part in the crystalline nanoporous δ form, are used as polymeric film.

Sensing elements for detection of organic pollutants can be prepared by deposition on substrates of polymers presenting a clathrate crystalline form. In particular, polymers in a clathrate form can be obtained by suitable solution processes, like solution casting, spin-coating, spray-coating or by solvent exposure of polymers obtained by melt processing, like extrusion or compression molding or injection molding. Suitable solvent are, for instance, halogenated compounds (chloroform, methylene chloride, carbon tetrachloride, dichloroethane, trichloroethylene, tetrachloroethylene, dibromoethane, methylene iodide, etc.) aromatic compounds (benzene, toluene, styrene, etc.) cyclic compounds (cyclohexane, tetrahydrofurane, etc.) as well as compounds containing sulfur atoms (carbon disulfide, etc.). Syndiotactic styrene homopolymer and copolymers in the crystalline nanoporous form δ, can be obtained from the polymers in a clathrate form by guest removal with volatile solvents, like acetone or methyl-ethyl-ketone, as described in IT 1271842, or by extraction procedures with carbon dioxide, as described in IT 1306004.

It is worth noting that manufacts based on the crystalline nanoporous form of syndiotactic styrene homopolymer and copolymers used as sensing elements, mainly films obtained by solution casting or spin-coating, can present some molecular orientation that is a crystallographic plane tend to be parallel to the film plane. This kind of orientation can be pointed out by large differences between the X-ray diffraction patterns obtained with the X-ray beam perpendicular or parallel to the film surface. Of course, in these cases, the ratio I(8.4)/I(10.6) on which is based the definition of the nanoporous δ form, cannot be evaluated with a standard automatic powder diffractometer, but the overall intensity of the two reflection presenting Miller indexes (010) and (-210) has to be collected by an automatic four circles diffractometer, for instance by collecting the pole figures for the two reflections.

The following examples are supplied in order to illustrate the invention without limiting the scope thereof.

### EXAMPLE 1

Syndiotactic polystyrene homopolymer was supplied by "Dow Chemical" under the trademark Questra 101. The percent of rrr tetrads evaluated by methylene region of ¹³C NMR spectra is of 92%. The molecular weight, determined by GPC analysis on trichlorobenzene, is 7x10⁵(polydispersity=3.3). Syndiotactic polystyrene samples were dissolved into chloroform (1% b.w. solutions) and then cast at room temperature on a silver electrode of a commercial AT-cut quartz disc, with a diameter of nearly 2 cm and thickness of 270 µm, presenting a main resonance frequency of 6 MHz. The obtained semicrystalline film, of a thickness close to 1 µm, presents a clathrate form including chloroform in amount close to 10% b.w. This clathrate form was transformed into the nanoporous δ form after treatment by carbon disulfide vapor at room temperature for 2 hours, followed by desiccation in a vacuum oven (pressure lower than 1 bar) at 45°C for 5 hours. The residual amount of volatile organic compounds, as measured by thermogravimetric analyses in the temperature range 40-200°C, was lower than 1 % b.w. The presence of the δ form in such film, was confirmed by X-ray diffraction measurements which indicate the presence of reflections of higher intensity at 2ϑ (CuKα) ≈ 8.4°, 10.6°, 13.3°, 16.8°, 20.7° 23.5°, and by an intensity ratio between two reflections presenting Miller indexes (010) and (-210), that is I(8.4)/I(10.6), larger than 10. Since these films present a substantial orientation of the crystalline phase this intensity ratio has been obtained by pole figures relative to both reflections.

The quartz frequencies were measured using a XTC/2 controller produced by Leybold Inficon (East Syracuse, NY, USA) which is able to detect resonance frequency variations of 0.05 Hz, thus allowing to measure mass variations of nearly 0.5 ng. This system is able to measure 4 frequency values per second using a reference oscillator at constant frequency.

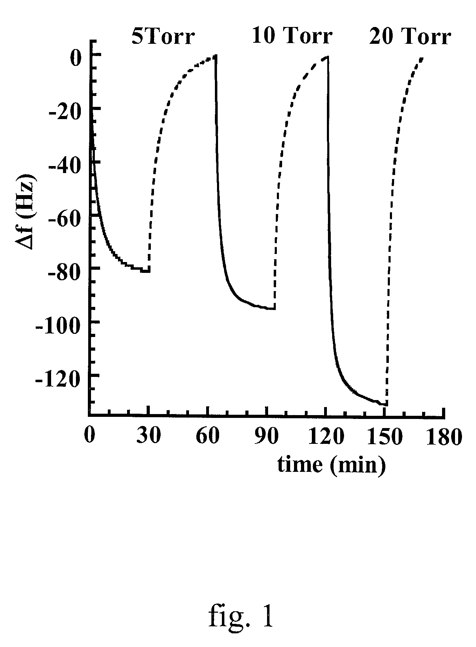

The response to chloroform of the prepared Quartz Crystal Microbalance (QCM) sensor, based on the δ form s-PS film, has been tested at 56°C in an apparatus allowing an accurate control of temperature (± 0.1 °C) and of the analyte pressure (± 0.005 torr). In particular, the frequency variation associated with instantaneous increases of chloroform pressure from zero to 5.7, 10 and 20 torr and with instantaneous decreases of chloroform pressure from these values to zero have been reported in Figure 1. It is worth noting that since the sensitivity of the apparatus used to measure the resonance frequency is 0.05 Hz, the prepared system can detect chloroform at least for pressures larger than 0.005 torr.

### EXAMPLE 2

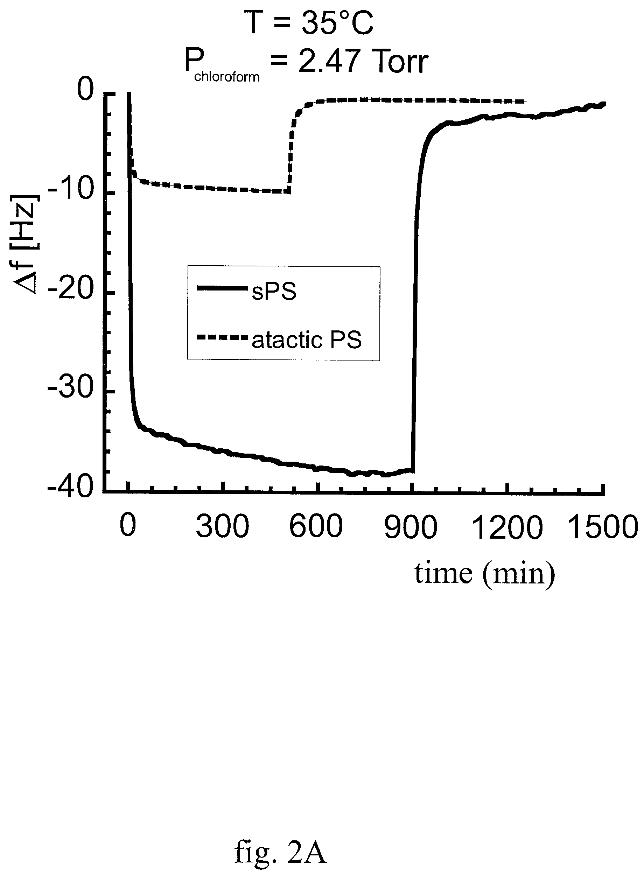

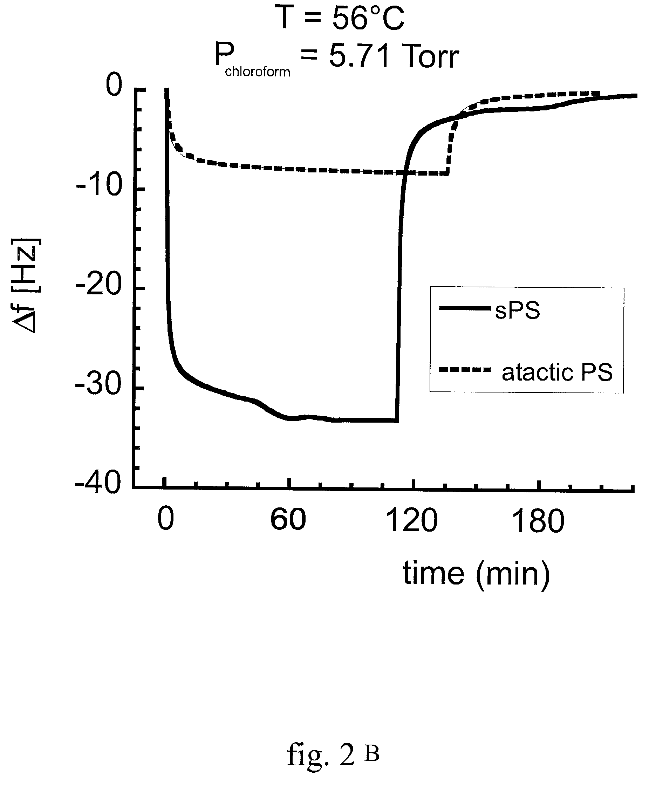

Syndiotactic polystyrene homopolymer of Example 1 was dissolved into chloroform (1% b.w. solutions) and then spray-coated at room temperature on an quartz disc like that one of Example 1. A QCM sensor was constructed by following the procedure described in Example 1. The response of the prepared QCM sensor at 35°C to sudden changes of chloroform pressure, from zero to 2.5 torr and viceversa, is shown in Figure 2A. The response of the prepared QCM sensor at 56°C to sudden changes of chloroform pressure, from zero to 5.7 torr and viceversa, is shown in Figure 2B

For the sake of comparison, an analogous QCM sensor based on a commercial atactic polystyrene homopolymer (a-PS) was constructed. In particular, the a-PS sample has been dissolved into chloroform (1% b.w. solutions) and then spray coated at room temperature on the silver electrode of the quartz disc, producing a film with a thickness close to 1µm. In order to remove the residual solvent, the a-PS film coated on the quartz crystal was directly desiccated in a vacuum oven at 45°C for 5 hours. The response of the a-PS based QCM sensor at 35°C to a sudden change of chloroform pressure, from zero to 2.5 torr and viceversa, is shown in Figure 2A. The response of the a-PS based QCM sensor at 56°C to a sudden change of chloroform pressure, from zero to 5.7 torr and viceversa, is shown in Figure 2B.

It is apparent that, for the considered temperature and chloroform pressure conditions, the sensitivity of the sensor based on the δ form s-PS is much higher than for the sensor based on a-PS.

## Claims

1. Use of syndiotactic polystyrene, or of syndiotactic copolymers of styrene, either at least in part in the crystalline nanoporous δ form, as sensing elements in sensors for the detection of organic pollutants.

2. Use according to claim 1, wherein said crystalline nanoporous δ form represents at least 80% of the crystalline fraction.

3. Use according to one of the preceding claims, wherein said crystalline nanoporous δ form shows an X-ray diffraction spectrum having maximum intensity reflections at 2ϑ (CuKα) ≈ 8.4°, 10.6°, 13.3°, 16.8°, 20.7° 23.5°, and an intensity ratio between two reflections presenting Miller indexes (010) and (-210), that is I(8.4)/I(10.6), larger than 5.

4. Use according to any one of the preceding claims, wherein said copolymers are styrene copolymers with comonomers belonging to the class formed by CH₂=CH-R olefins, where R is an alkyl-aryl or an halogen-aryl radical with 6-20 carbon atoms, and by ethylenically unsaturated monomers.

5. Use according to anyone of the preceding claims, wherein in said copolymers of styrene said comonomers are present in an amount lower than 80% by mol.

6. Sensors for detection of organic pollutants including sensing elements comprising syndiotactic polystyrene or syndiotactic copolymers of styrene, either at least in part in the crystalline nanoporous δ form according to anyone of the preceding claims.

7. Sensors according to claim 6, wherein the sensors are selected from the class formed by bulk acoustic wave (BAW) sensors, surface acoustic wave (SAW) sensors and flexural plate wave (FPW) sensors.

8. Sensors according to claims 6 or 7,wherein a commercial quartz crystal microbalance (QCM) is used.

9. A process for the preparation of sensing elements to be used in sensors for the detection of organic pollutants through deposition on the sensor substrate of a polymeric film, **characterised by** the fact that syndiotactic polystyrene or syndiotactic copolymers of styrene, either at least in part in the crystalline nanoporous δ form, are used as polymeric film.
